# FASCICULE DE BREVET EUROPEEN

(11) **EP 3 024 416 B1**
(45) Date de publication et mention de la délivrance du brevet: **10.05.2017**
(21) Numéro de dépôt: 14750577.0
(22) Date de dépôt: 24.07.2014
(51) Int. Cl.: A61F 2/00

(54) **PROTHÈSE IMPLANTABLE**
IMPLANTIERBARE PROTHESE
IMPLANTABLE PROSTHESIS

(30) Priorité: 25.07.2013 FR 1357362
(43) Date de publication de la demande: 01.06.2016
(73) Titulaire: Cousin Biotech, 59117 Wervicq Sud (FR)
(72) Inventeur: SOLECKI, Gilles, 59390 Lannoy (FR); KAMCHE, Farid, 59200 Tourcoing (FR); NOEL, Stéphane, 59496 Hantay (FR)
(74) Mandataire: Balesta, Pierre
(86) Numéro de dépôt international: PCT/FR2014/051917
(87) Numéro de publication internationale: WO 2015/011417

(56) Documents cités:
- EP-A1- 1 336 391
- WO-A1-02/22047
- GB-A- 2 430 372
- US-A1- 2003 212 462

## Description

### Arrière-plan de l'invention

La présente invention a trait au domaine des dispositifs médicaux implantables.

Plus précisément, l'invention concerne une prothèse implantable qui comporte :
- une partie adhérente pouvant former des adhérences avec des tissus organiques, la partie adhérente présentant un premier bord périphérique ;
- une partie non-adhérente, inhibant la formation d'adhérences avec des tissus organiques, solidaire de la partie adhérente et présentant un second bord périphérique, le premier bord périphérique étant en retrait du second bord périphérique de manière à découvrir partiellement la partie non-adhérente en définissant, entre le premier bord périphérique et le second bord périphérique, une bande périphérique non-adhérente qui entoure la partie adhérente.

Une telle prothèse est notamment implantée dans la cavité péritonéale afin de réparer la paroi abdominale.

Traditionnellement, la partie adhérente est destinée à être placée contre le péritoine pour être colonisée par les tissus humains, tandis que la partie non-adhérente est configurée pour limiter les adhérences qui se produisent entre la prothèse et les organes abdominaux après l'intervention chirurgicale.

WO 99/06079 décrit une telle prothèse composite de forme rectangulaire dans laquelle la partie non-adhérente est constituée d'un film de polysaccharide de forme rectangulaire qui déborde latéralement de plusieurs millimètres de la partie adhérente réalisée en tricot poreux, et ce afin d'éviter les contacts entre les viscères et le bord périphériques de la partie adhérente.

Cette prothèse ne permet toutefois pas d'éviter les adhérences au niveau du bord périphérique de la partie adhérente. Ces adhérences apparaissent car il n'est pas possible de mettre en contact, de manière uniforme, la partie adhérente plane avec la surface concave du péritoine. Des plis se forment dans la prothèse, ce qui a pour effet de créer des zones adhérentes libres dans lesquelles la partie adhérente ne vient pas en contact du péritoine. Il arrive donc que les viscères viennent adhérer sur ces zones adhérentes libres, ce qui est de nature à compliquer la cicatrisation du péritoine.

GB 2430372 décrit une prothèse implantable ayant une partie adhérente constituée d'une pluralité de portions adhérentes disjointes les unes des autres qui s'étendent radialement autour d'un manchon.

US 2003/0212462 décrit une prothèse implantable ayant une partie adhérente en forme de coeur.

### Objet et résumé de l'invention

Un objet de l'invention est de proposer une prothèse implantable qui remédie aux inconvénients précités, et dans laquelle on diminue encore le risque de formation d'adhérence entre les viscères et la partie adhérente.

L'invention atteint son but par le fait que la partie adhérente a une partie centrale et présente une forme en étoile qui comprend une pluralité de branches qui s'étendent depuis la partie centrale de la partie adhérente, la partie adhérente comportant une pluralité d'échancrures, chaque échancrure étant située entre deux branches adjacentes.

Grâce à sa forme en étoile, la partie adhérente épouse mieux la surface concave du péritoine, ce qui a pour effet de diminuer sensiblement, voire éviter la formation de zones d'adhérence libres.

Dit autrement, les branches se déforment pour épouser la forme concave du péritoine. Les branches étant en contact avec le péritoine, la partie adhérente reste bien en contact avec le péritoine, et notamment le long du bord périphérique de la partie adhérente. A ce titre, on comprend que les parties adhérente et non-adhérente sont souples.

On comprend par ailleurs que la partie non-adhérente déborde également latéralement de la périphérie de la partie adhérente, dès lors que le premier bord périphérique de la partie adhérente est en retrait du second bord périphérique.

Les branches de la partie adhérente sont jointives les unes aux autres par le fait qu'elles s'étendent depuis la partie centrale de la partie adhérente.

De préférence, la largeur minimale de la bande périphérique non-adhérente est comprise entre 3 mm et 6 mm

De préférence, le nombre de branches est compris entre quatre et dix, de préférence six.

Avantageusement, chaque branche s'étend radialement selon une hauteur et s'étend en pointe depuis la partie centrale de la portion adhérente vers son sommet. De préférence, le sommet est arrondi. Encore de préférence, la base de la branche est plus large que son sommet.

Les branches présentent préférentiellement la même hauteur. Toutefois, sans sortir du cadre de la présente invention, certaines des baleines pourraient présenter des hauteurs supérieures aux autres.

De manière préférentielle, les branches présentent une hauteur comprise entre 3 mm et 50 mm. Une telle gamme de hauteurs permet une bonne conformation des branches, et de la partie adhérente, au péritoine. De préférence, la largeur de la bande périphérique non-adhérente est comprise entre 3 mm et 10 mm au niveau du sommet des branches.

Avantageusement, la partie centrale présente une envergure comprise de préférence entre 40 mm et 90 mm.

Selon un mode de réalisation avantageux, les branches forment une seule pièce avec la partie centrale. Pour ce faire, la partie adhérente est par exemple obtenue en découpant un tricot poreux pour lui donner une forme en étoile.

Selon une variante, les branches sont des pièces rapportées qui sont fixées à la partie centrale.

Avantageusement, la partie centrale présente une forme circulaire ou ovale. Le rayon de la partie centrale est préférentiellement supérieur ou égal à la hauteur des branches de la partie adhérente.

De préférence, la partie adhérente est réalisée dans une matière textile. Il s'agit par exemple, mais non exclusivement d'un tricot de mailles jetées de type Chaine ou Raschel. La partie adhérente est préférentiellement constituée, au moins en partie, de PLLA.

Selon une variante, les branches sont réalisées dans une matière textile plus rigide que la matière textile constituant la partie centrale.

Dans un mode de réalisation, la partie non-adhérente présente la forme d'un disque ou d'un ovale.

Cette forme en disque ou ovale permet de mieux s'adapter à la forme concave du péritoine que la forme rectangulaire de l'art antérieure.

Selon un autre mode de réalisation particulièrement avantageux, la partie non-adhérente présente également une forme en étoile, et les branches de la partie non-adhérente sont superposées avec des branches de la partie adhérente.

Ainsi, la partie adhérente comporte au moins autant de branches que la partie non-adhérente.

Un intérêt de la forme en étoile de la partie non-adhérente est d'améliorer encore la conformation de la prothèse à la forme concave du péritoine. On évite en outre que la partie non-adhérente ne forme des plis entre les branches de la partie adhérente, où pourraient s'amasser des tissus organiques.

Là-encore, les branches de la partie non-adhérente débordent latéralement des branches de la partie adhérente de façon à définir la bande périphérique non-adhérente. De préférence, la largeur minimale de la bande périphérique non-adhérente est comprise entre 5 mm et 15 mm entre deux branches adjacentes.

On comprend donc que, dans ce mode de réalisation avantageux, la prothèse implantable présente la forme d'une étoile.

Avantageusement, la partie non-adhérente comporte une portion centrale d'où s'étendent en pointe les branches de la partie non-adhérente.

Encore de préférence, les sommets des branches de la partie non-adhérente sont arrondis.

Selon une variante avantageuse, la partie adhérente est réalisée en tricot, tandis que la partie non-adhérente est un film polymère. De préférence, mais non exclusivement, le film polymère est un polymère résorbable d'acide lactique.

Le film est fixé au tricot par couture ou fusion des microfilaments sur le fil.

Pour améliorer le positionnement de la prothèse dans le corps lors de l'intervention, elle comporte en outre un moyen d'expansion amovible. Ce moyen d'expansion est par exemple un ballonnet gonflable que le chirurgien peut gonfler par l'intermédiaire d'un tube. Ce ballonnet gonflable est de préférence logé dans une poche fixée à la partie adhérente. La poche présente de préférence la forme d'un disque épais et comprend une paroi en tricot fixée à la partie adhérente et munie d'un orifice permettant le retrait du ballonnet après l'implantation de la prothèse.

La prothèse est plane ou, dans une variante avantageuse, présente une forme bombée afin d'épouser encore mieux la forme concave du péritoine.

### Brève description des dessins

L'invention sera mieux comprise à la lecture de la description qui suit de modes de réalisation de l'invention donnés à titre d'exemples non limitatifs, en référence aux dessins annexés, sur lesquels :
- la figure **1** illustre un premier mode de réalisation d'une prothèse implantable selon l'invention, en vue de dessus, dans laquelle la partie adhérente présente une étoile à six branches, tandis que la partie non-adhérente présente la forme d'un disque ;
- la figure **2** représente la prothèse de la figure **1** en vue de côté ;
- la figure **3** illustre la prothèse de la figure **1** positionnée au contact du péritoine ;
- la figure **4** illustre un deuxième mode de réalisation d'une prothèse implantable selon l'invention, en vue de dessus, dans laquelle la partie non-adhérente présente également la forme d'une étoile à six branches ;
- la figure **5** représente la prothèse de la figure **4** en vue de côté ;
- la figure **6** illustre une variante de la prothèse de la figure **4** dans laquelle les branches de la partie adhérente sont rapportées ;
- la figure **7** illustre une variante de la prothèse de la figure **4**, dans laquelle les parties adhérente et non-adhérente comportent chacune huit branches ; et
- les figures **8** et **9** représentent un autre mode de réalisation de la prothèse de la figure **4** qui comporte en outre un ballonnet d'expansion.

### Description détaillée de l'invention

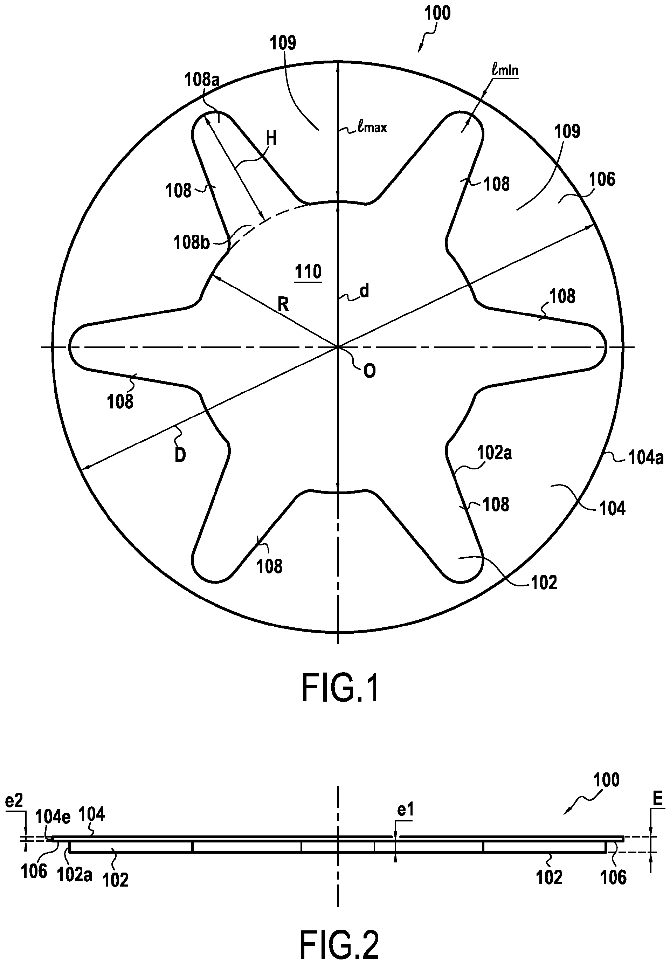

A l'aide des figures **1** à **3**, on va décrire un premier mode de réalisation d'une prothèse implantable **100** conforme à la présente invention.

Dans cet exemple, la prothèse implantable **100** est une prothèse intra-péritonéale. Ce type de prothèse est également appelée plaque viscérale intra-péritonéale. La prothèse est souple afin de venir se conformer à la concavité du péritoine.

La figure **1** illustre en vue de dessus la prothèse implantable **100.** Cette dernière présente la forme générale d'un disque de centre **O** dont le diamètre **D** est de l'ordre de 100 mm. La prothèse **100** présente une épaisseur **E** de l'ordre de 500 microns.

La prothèse **100** est du type composite et comporte une partie adhérente **102** solidaire d'une partie non-adhérente **104.**

De manière traditionnelle, la partie adhérente **102** est conçue pour former des adhérences avec des tissus organiques, tandis que la partie non-adhérente **104** est conçue pour inhiber la formation d'adhérences avec des tissus organiques.

Après avoir été implantée dans le corps humain, la partie adhérente **102** vient en contact du péritoine afin d'y adhérer, tandis que la partie non-adhérente **104** forme une barrière qui vient au contact des viscères afin d'empêcher ces dernières d'adhérer avec le péritoine pendant la période de cicatrisation.

Dans cet exemple, la partie adhérente est un tricot de mailles jetées de type Chaine ou Raschel. De préférence, le tricot comporte une partie résorbable et une partie non résorbable. Selon une variante, le tricot pourrait être entièrement non résorbable.

Dans cet exemple, le tricot comporte une partie résorbable réalisée avec un monofilament d'environ 200 dtex en PLLA, et une partie non résorbable réalisée avec un monofilament d'environ 70 dtex en polypropylène.

Dans ce premier mode de réalisation, la partie non-adhérente **104** est film polymère résorbable d'acide lactique. Le film polymère est fixé au tricot par couture ou fusion des monofilaments sur le film.

Dans cet exemple, la partie adhérente **102** présente une épaisseur e1 de l'ordre de 475 microns tandis que la partie non-adhérente **104** présente une épaisseur **e2** de l'ordre de 25 microns.

En se référant à nouveau à la figure **1**, on constate que la partie adhérente **102** présente un premier bord périphérique **102**a. La partie non-adhérente **104**, en forme de disque dans cet exemple, comporte quant à elle un second bord périphérique **104**a qui est circulaire.

Comme on le constate sur les figures **1** et **2**, le premier bord périphérique **102**a de la partie adhérente **102** est en retrait du second bord périphérique **104**a de la partie non-adhérente **104** de manière à découvrir partiellement la partie adhérente en définissant, entre le premier bord périphérique **102**a et le second bord périphérique **104**a, une bande périphérique non-adhérente **106** qui entoure la partie adhérente **102**.

A l'aide de la figure **2**, on conçoit que la bande périphérique non-adhérente **106** est dans le même plan que la partie non-adhérente **104,** et présente la même épaisseur **e2.**

On constate également que la bande périphérique non-adhérente **106** ne recouvre pas la périphérie de la partie adhérente **102.**

La bande périphérique non-adhérente **106,** définie entre le premier bord périphérique **102**a et le second bord périphérique **104**a, présente une largeur **I** qui est définie entre le premier bord périphérique **102**a et le second bord périphérique **104**a.

Sur la figure **1**, on appelle **Imin,** la largeur radiale minimale de la bande périphérique non-adhérente **106** et **Imax,** la largeur maximale de la bande périphérique non-adhérente **106.**

Conformément à l'invention, la partie adhérente **102** présente une forme en étoile qui comprend une pluralité de branches **108**. Dans cet exemple, la partie adhérente comporte six branches **108**. Chacune des branches **108** s'étend radialement depuis une partie centrale **110** de la partie adhérente.

La partie adhérente comporte en outre une pluralité d'échancrures **109**, chacune des échancrures **109** étant située entre deux branches adjacentes.

Les branches sont jointives les unes aux autres grâce au fait qu'elles s'étendent depuis la partie centrale **110** de la partie adhérente. La partie adhérente s'étend donc continûment entre les différentes branches. Chaque branche **108** s'étend radialement selon une hauteur **H** et présente un sommet **108**a, ainsi qu'une base **108b**. Chaque branche s'étend en pointe depuis la partie centrale vers son sommet. Dans cet exemple, les branches présentent la même hauteur **H**, de l'ordre de **20** mm. La base de chaque branche est plus large que son sommet. Chaque branche comporte ainsi une partie convergente vers son sommet. En outre, on constate que les sommets des branches sont arrondis.

Le diamètre **d** de la partie centrale **110** où s'étendent les branches présente la forme générale d'un disque dont le diamètre est de l'ordre de **60** mm dans cet exemple.

On constate sur la figure **1** que le centre de la partie non-adhérente en forme de disque est sensiblement confondu avec le centre de la partie centrale de forme circulaire. Par ailleurs, la prothèse présente une symétrie centrale de centre **O.**

A l'aide de la figure **1**, on constate par ailleurs que la largeur minimale **Imin** de la bande périphérique non-adhérente **106** est située au niveau du sommet **108**a des branches **108**. Dans cet exemple, la largeur **Imin**, correspondant au retrait minimal, est de l'ordre de 5 mm.

On constate également que la largeur maximale **Imax**, correspondant au retrait maximal, est située entre deux branches consécutives et de l'ordre de 40 mm.

Dans l'exemple de la figure **1**, les branches **108** forment une seule pièce avec la partie centrale **110**.

On constate par ailleurs que le rayon **R** de la partie centrale est légèrement supérieur à la hauteur **H** des branches de la partie adhérente. Aussi, les branches présentent des dimensions petites par rapport à celles de la partie centrale.

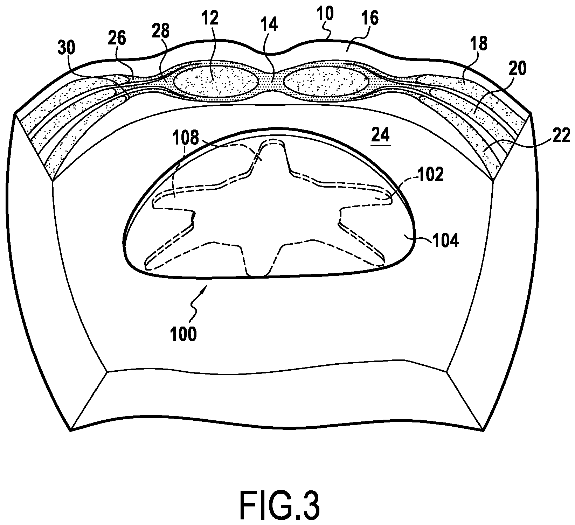

En se référant à la figure **3**, on va maintenant expliquer le positionnement de la prothèse **100** dans la zone péritonéale du patient.

Cette figure représente de façon schématique une portion de l'abdomen, selon une section prise au-dessus du nombril.

Sur la figure **3**, les références suivantes sont utilisées :
- **10** :: peau ;
- **12** :: muscle droit de l'abdomen ;
- **14** :: ligne blanche ;
- **16** :: tissu sous cutané (couche graisseuse) ;
- **18** :: muscle oblique externe ;
- **20** :: muscle oblique interne ;
- **22** :: muscle transverse de l'abdomen ;
- **24** :: péritoine ;
- **26** :: aponévrose du muscle oblique externe ;
- **28** :: aponévrose du muscle oblique interne ;
- **30** :: aponévrose du muscle transverse de l'abdomen.

Comme on le constate sur la figure **3**, la prothèse adopte la concavité du péritoine **24** et reste uniformément en contact avec le péritoine après son placement sur le site chirurgical.

De manière classique, pour sa mise en place, la prothèse prend la forme d'un parapluie lors de son transfert à l'intérieur du péritoine par le chirurgien, puis se redéploie pour venir au contact du péritoine.

La prothèse de l'exemple de la figure **3** vise en particulier à soigner les hernies de la ligne blanche en regard des deux muscles droits de la paroi abdominale ou bien encore les hernies ombilicales.

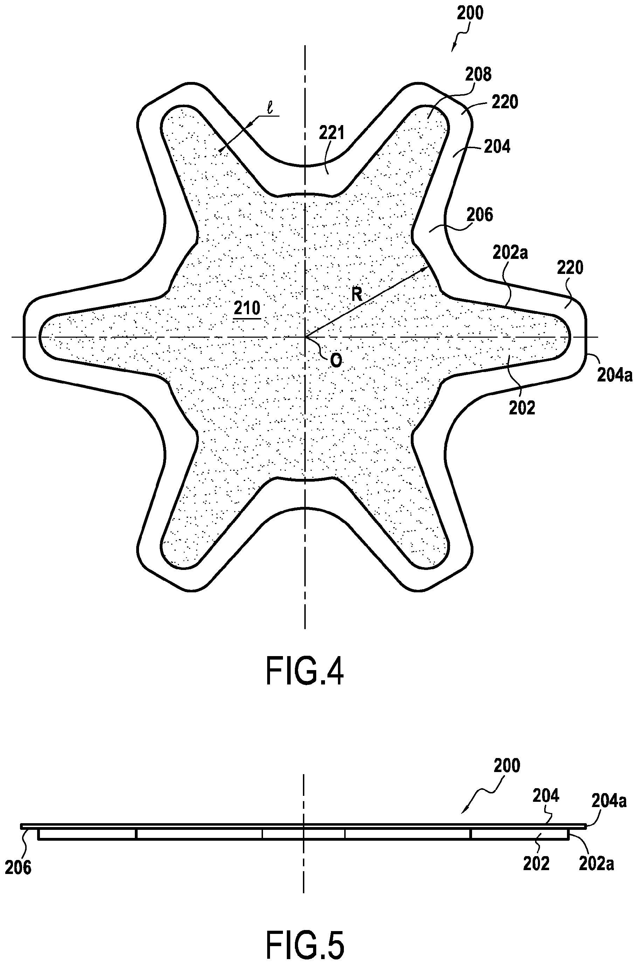

A l'aide des figures **4** et **5**, on va maintenant décrire un deuxième mode de réalisation de la prothèse **200** selon l'invention.

Le deuxième mode de réalisation de la prothèse implantable selon la présente invention diffère du premier par le fait que la partie non-adhérente **204** présente également une forme en étoile.

Dans l'exemple de la figure **4**, la partie non-adhérente **204** présente six branches **220** qui sont superposées avec les branches **208** de la partie adhérente. La partie non-adhérente comporte une portion centrale **221** d'où s'étendent radialement en pointe les branches **220** de la partie non-adhérente. Les branches de la partie non-adhérente s'étendent en pointe vers leurs sommets respectifs, ces derniers étant arrondis.

On constate par ailleurs que la partie périphérique non-adhérente **206** qui entoure la partie adhérente **202** présente une largeur **I**, de l'ordre de 5 mm, qui est sensiblement constante selon tout le pourtour de la prothèse **200**.

On rappelle que la partie périphérique non-adhérente **206** est définie entre le premier bord périphérique **202**a de la partie adhérente **202** et le second bord périphérique **204**a de la partie non-adhérente **204**.

Dans l'exemple de réalisation de la figure **4**, la largeur **I** de la bande périphérique non-adhérente est comprise entre 3 et 10 mm, et ce sur tout le pourtour de la prothèse.

Dans le deuxième mode de réalisation, les branches **208** de la partie adhérente **202** forment une seule pièce avec la partie centrale **210**.

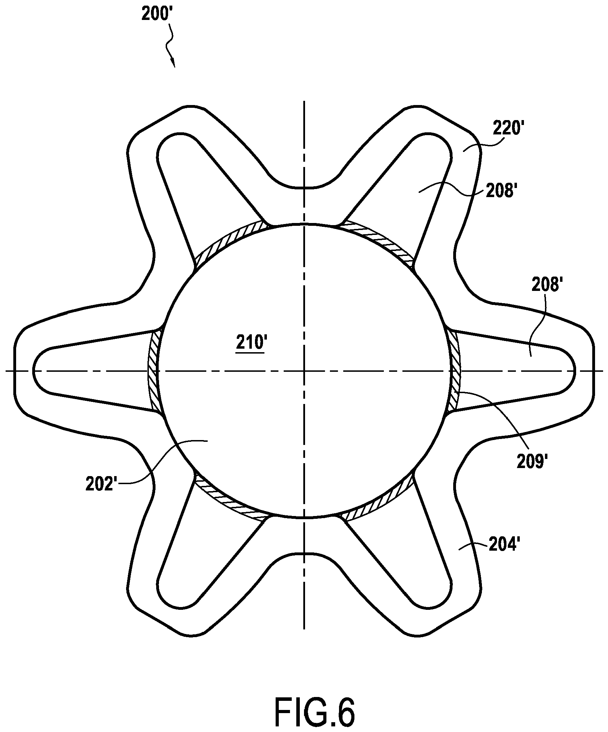

Dans la variante de la figure **6**, les branches **208'** de la partie adhérente **202** sont des pièces rapportées qui sont fixées à la partie centrale **210'**. Les branches **208'** de la partie adhérente **202** sont réalisées dans une matière textile qui est plus rigide que la matière textile constituant la partie centrale **210'**. Dans l'exemple de la figure **6****,** les branches **208'** sont soudées, par l'intermédiaire de soudures **209'** à la partie centrale **210'**. On pourrait toutefois envisager une autre moyen de fixation sans sortir du cadre de la présente invention.

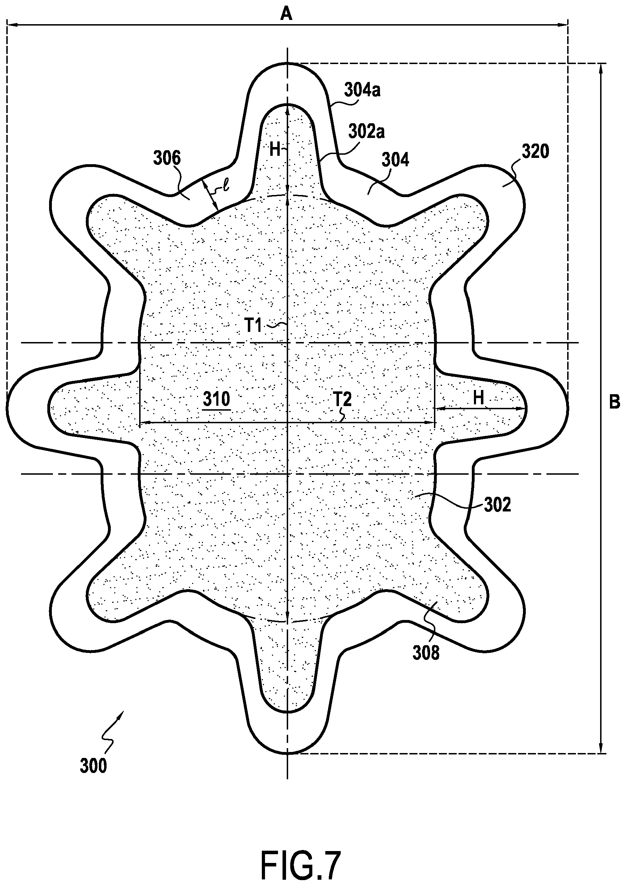

La prothèse implantable **300** illustrée sur la figure **7** est une variante de celle de la figure **4**.

Dans cette variante, la partie centrale **310** de la partie adhérente **302** présente une forme sensiblement ovale. Ces dimensions longitudinale et transversale sont référencées **T1** et **T2** sur la figure **7**. On constate que la demi-largeur de la partie centrale, considérée selon la dimension transversale **T2**, est supérieure à la hauteur **H** des branches. Aussi, les branches présentent là-encore des dimensions petites par rapport à celles de la partie centrale.

Par ailleurs, la partie adhérente **302** comporte huit branches **308**. La partie non-adhérente **304** comporte quant à elle également huit branches **320**. La largeur **A** de la prothèse **300** est de l'ordre de 125 mm, tandis que la longueur **B** est de l'ordre de 155 mm.

Bien évidemment, d'autres formes en étoile des parties adhérentes et non-adhérentes pourraient être envisagées, ainsi qu'un nombre différent de branches.

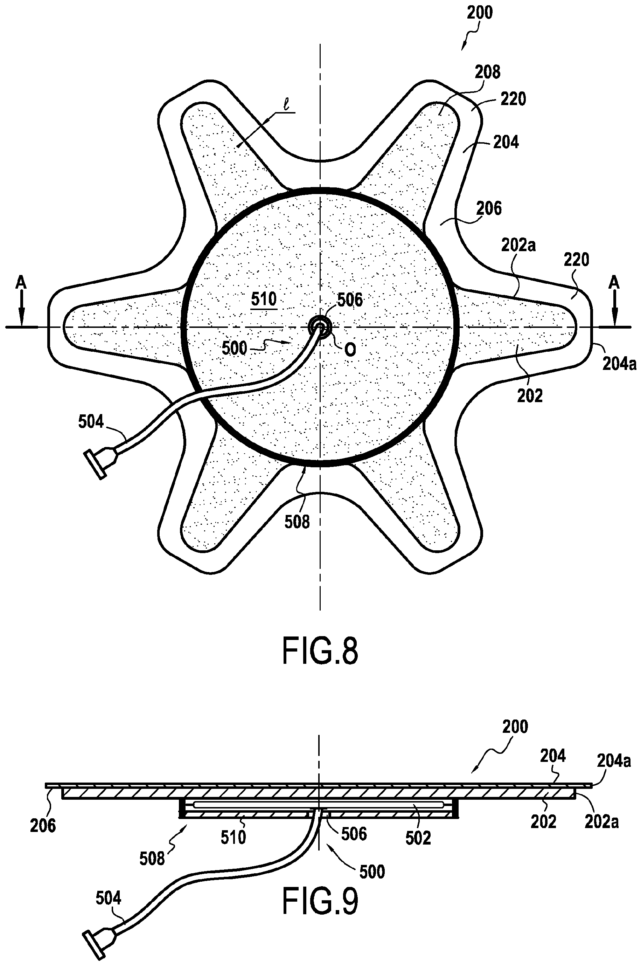

Dans le mode de réalisation des figures **8** et **9**, la prothèse **200** comporte en outre un moyen d'expansion **500** amovible, qui se présente sous la forme d'un ballonnet gonflable **502**.

Le ballonnet **502** est relié à l'extrémité d'un tube de gonflage **504**. A l'état dégonflé, le ballonnet présente la forme d'une pastille souple qui est prévue pour être introduite par un orifice **506** à l'intérieur d'une poche **508** fixée à la partie adhérente **202** de la prothèse **200**. La poche **508** comporte une paroi extérieure **510** fixée à la partie adhérente **202**. Dans cet exemple, la paroi extérieure **510** est réalisée en tricot et est cousue au tricot de la partie adhérente. On pourrait toutefois prévoir une fixation par soudure. Comme on le constate sur la figure **8**, dans cet exemple, la paroi extérieure **510** présente la forme d'un disque inscrit à l'intérieur de la partie adhérente en forme d'étoile **202.**

Lorsque l'on pompe un fluide dans le tube **504,** le ballonnet se gonfle, ce qui a pour effet de donner une forme bombée à la prothèse **200**, et d'améliorer sa conformation à la concavité du péritoine. Après implantation de la prothèse, le ballonnet est évacué par l'orifice **506** après avoir été préalablement dégonflé.

## Revendications

1. Prothèse implantable (100, 200, 200', 300) comportant :
une partie adhérente (102, 202, 202', 302) pouvant former des adhérences avec des tissus organiques, la partie adhérente présentant un premier bord périphérique (102a, 202a, 302a) ;
une partie non-adhérente (104, 204, 204', 304), inhibant la formation d'adhérences avec des tissus, solidaire de la partie adhérente et présentant un second bord périphérique (104a, 204a, 304a), le premier bord périphérique (102a) étant en retrait du second bord périphérique (104a) de manière à découvrir partiellement la partie non-adhérente en définissant, entre le premier bord périphérique et le second bord périphérique, une bande périphérique non-adhérente (106) qui entoure la partie adhérente (102) ;
la partie adhérente (102, 202, 202', 302) ayant une partie centrale (110, 210, 210', 310) et présentant une forme en étoile qui comprend une pluralité de branches (108, 208, 208', 308) qui s'étendent depuis la partie centrale de la partie adhérente, la partie adhérente comportant une pluralité d'échancrures (109), chaque échancrure étant située entre deux branches adjacentes.

2. Prothèse selon la revendication **1**, dans laquelle chaque branche (108) s'étend radialement selon une hauteur (H), et s'étend en pointe depuis la partie centrale de la portion adhérente vers son sommet (108a).

3. Prothèse selon la revendication **2**, dans laquelle les branches présentent une hauteur (H) comprise entre 3 mm et 50 mm.

4. Prothèse selon la revendication **2** ou **3**, dans laquelle la largeur (I) de la bande périphérique non-adhérente est comprise entre 3 mm et 10 mm au niveau du sommet (108a) des branches (108).

5. Prothèse selon l'une quelconque des revendications **1** à **4**, dans laquelle les branches (108, 208, 308) forment une seule pièce avec la partie centrale (110, 210, 310).

6. Prothèse selon l'une quelconque des revendications **1** à **4**, dans laquelle les branches (208') sont des pièces rapportées qui sont fixées à la partie centrale (210').

7. Prothèse selon l'une quelconque des revendications **1** à **6**, dans laquelle la partie centrale (110, 210, 210', 310) présente une forme circulaire ou ovale.

8. Prothèse selon la selon quelconque des revendications **1** à **7**, dans laquelle le nombre de branches (108, 208, 208', 308) est compris entre quatre et dix.

9. Prothèse selon l'une quelconque des revendications **1** à **8**, dans laquelle la partie adhérente (102, 202, 202', 302) est réalisée dans une matière textile.

10. Prothèse selon les revendications **6** et **9**, dans laquelle les branches (208') sont réalisées dans une matière textile plus rigide que la matière textile constituant la partie centrale (210').

11. Prothèse selon l'une quelconque des revendications **1** à **10**, dans laquelle la partie non-adhérente (104, 204, 204', 304) présente la forme d'un disque ou d'un ovale.

12. Prothèse selon l'une quelconque des revendications **1** à **10**, dans laquelle la partie non-adhérente (204, 204', 304) présente également une forme en étoile, et dans laquelle les branches (220) de la partie non-adhérente sont superposées avec des branches (208, 208', 308) de la partie adhérente.

13. Prothèse selon la revendication **12**, dans laquelle la partie non-adhérente comporte une portion centrale (221) d'où s'étendent en pointe les branches (220) de la partie non-adhérente.

14. Prothèse selon l'une quelconque des revendications **1** à **13**, dans laquelle la partie adhérente (102, 202, 202', 302) est réalisée en tricot, tandis que la partie non-adhérente est un film polymère.

15. Prothèse selon l'une quelconque des revendications **1** à **14**, comportant en outre un moyen d'expansion amovible (500).

## Patentansprüche

1. Implantierbare Prothese (100, 200, 200', 300), umfassend:
einen haftenden Teil (102, 202, 202', 302), der Haftverbindungen mit organischen Geweben bilden kann, wobei der haftende Teil einen ersten Umfangsrand (102a, 202a, 302a) aufweist,
einen nicht-haftenden Teil (104, 204, 204', 304), der die Bildung von Haftverbindungen mit Geweben inhibiert, mit dem haftenden Teil fest verbunden ist und einen zweiten Umfangsrand (104a, 204a, 304a) aufweist, wobei der erste Umfangsrand (102a) von dem zweiten Umfangsrand (104a) derart versetzt ist, dass der nicht-haftende Teil teilweise freigelegt wird, indem, zwischen dem ersten Umfangsrand und dem zweiten Umfangsrand, eine nicht-haftende Umfangsfläche (106) definiert wird, die den haftenden Teil (102) umgibt,
wobei der haftende Teil (102, 202, 202', 302) einen zentralen Teil (110, 210, 210`, 310) aufweist und eine Form eines Sterns hat, der mehrere Strahlen (108, 208, 208', 308) umfasst, die sich von dem zentralen Teil des haftenden Teils aus erstrecken, wobei der haftende Teil mehrere Ausnehmungen (109) umfasst, wobei jede Ausnehmung zwischen zwei benachbarten Strahlen angeordnet ist.

2. Prothese gemäß Anspruch 1, wobei sich jeder Strahl (108) radial in einer Höhe (H) erstreckt, und sich spitz zulaufend von dem zentralen Teil des haftenden Teils zu seiner Spitze (108a) erstreckt.

3. Prothese gemäß Anspruch 2, wobei die Strahlen eine Höhe (H) zwischen 3 mm und 50 mm aufweisen.

4. Prothese gemäß Anspruch 2 oder 3, wobei die Breite (I) der nicht-haftenden Umfangsfläche zwischen 3 mm und 10 mm auf der Höhe der Spitze (108a) der Strahlen (108) liegt.

5. Prothese gemäß einem der Ansprüche 1 bis 4, wobei die Strahlen (108, 208, 308) ein einziges Stück mit dem zentralen Teil (110, 210, 310) bilden.

6. Prothese gemäß einem der Ansprüche 1 bis 4, wobei die Strahlen (208') angesetzte Stücke sind, die an dem zentralen Teil (210') befestigt sind.

7. Prothese gemäß einem der Ansprüche 1 bis 6, wobei der zentrale Teil (110, 210, 210`, 310) eine Kreisform oder ovale Form aufweist.

8. Prothese gemäß einem der Ansprüche 1 bis 7, wobei die Anzahl der Strahlen (108, 208, 208', 308) zwischen vier und zehn liegt.

9. Prothese gemäß einem der Ansprüche 1 bis 8, wobei der haftende Teil (102, 202, 202', 302) aus einem Textilmaterial hergestellt ist.

10. Prothese gemäß den Ansprüchen 6 und 9, wobei die Strahlen (208') aus einem steiferen Textilmaterial hergestellt sind als das Textilmaterial, das den zentralen Teil (210') bildet.

11. Prothese gemäß einem der Ansprüche 1 bis 10, wobei der nicht-haftende Teil (104, 204, 204', 304) die Form einer Scheibe oder eines Ovals aufweist.

12. Prothese gemäß einem der Ansprüche 1 bis 10, wobei der nicht-haftende Teil (204, 204', 304) auch eine Sternform aufweist, und wobei die Strahlen (220) des nicht-haftenden Teils von den Strahlen (208', 208', 308) des haftenden Teils überlagert werden.

13. Prothese gemäß Anspruch 12, wobei der nicht-haftende Teil einen zentralen Abschnitt (221) umfasst, von dem aus sich spitz zulaufend die Strahlen (220) des nicht-haftenden Teils erstrecken.

14. Prothese gemäß einem der Ansprüche 1 bis 13, wobei der haftende Teil (102, 202, 202', 302) aus einem Gewebe hergestellt ist, während der nicht-haftende Teil ein Polymerfilm ist.

15. Prothese gemäß einem der Ansprüche 1 bis 14, ferner umfassend ein abnehmbares Expansionsmittel (500).

## Claims

1. An implantable prosthesis (100, 200, 200', 300) comprising:
· an adhesive portion (102, 202, 202', 302) that is capable of forming adhesions with organic tissues, the adhesive portion presenting a first peripheral edge (102a, 202a, 302a);
· a non-adhesive portion (104, 204, 204', 304) that inhibits the formation of adhesions with tissues, that is secured to the adhesive portion, and that presents a second peripheral edge (104a, 204a, 304a), the first peripheral edge (102a) being set back from the second peripheral edge (104a) so as to uncover the non-adhesive portion, in part, defining, between the first peripheral edge and the second peripheral edge, a non-adhesive peripheral band (106) that surrounds the adhesive portion (102);
the adhesive portion (102, 202, 202', 302) having a central portion (110, 210, 210', 310), and presenting the shape of a star that includes a plurality of branches (108, 208, 208', 308) that extend from the central portion of the adhesive portion, the adhesive portion including a plurality of notches (109), each notch being situated between two adjacent branches.

2. A prosthesis according to claim 1, wherein each branch (108) extends radially over a height (H), and it tapers from the central portion of the adhesive portion towards its vertex (108a).

3. A prosthesis according to claim 2, wherein the branches present a height (H) that lies in the range 3 mm to 50 mm.

4. A prosthesis according to claim 2 or claim 3, wherein the width (ℓ) of the non-adhesive peripheral band lies in the range 3 mm to 10 mm at the vertices (108a) of the branches (108).

5. A prosthesis according to any one of claims 1 to 4, wherein the branches (108, 208, 308) are formed integrally with the central portion (110, 210, 310).

6. A prosthesis according to any one of claims 1 to 4, wherein the branches (208') are separate pieces that are fastened to the central portion (210').

7. A prosthesis according to any one of claims 1 to 6, wherein the central portion (110, 210, 210', 310) presents a shape that is circular or oval.

8. A prosthesis according to any one of claims 1 to 7, wherein the number of branches (108, 208, 208', 308) lies in the range four to ten.

9. A prosthesis according to any one of claims 1 to 8, wherein the adhesive portion (102, 202, 202', 302) is made out of a textile material.

10. A prosthesis according to claim 6 and claim 9, wherein the branches (208') are made out of a textile material that is more rigid than the textile material that constitutes the central portion (210').

11. A prosthesis according to any one of claims 1 to 10, wherein the non-adhesive portion (104, 204, 204', 304) presents the shape of a disk or of an oval.

12. A prosthesis according to any one of claims 1 to 10, wherein the non-adhesive portion (204, 204', 304) also presents the shape of a star, and wherein the branches (220) of the non-adhesive portion are superposed with branches (208, 208', 308) of the adhesive portion.

13. A prosthesis according to claim 12, wherein the non-adhesive portion includes a central portion (221) from where the branches (220) of the non-adhesive portion taper.

14. A prosthesis according to any one of claims 1 to 13, wherein the adhesive portion (102, 202, 202', 302) is made out of knitted fabric, while the non-adhesive portion is a polymer film.

15. A prosthesis according to any one of claims 1 to 14, further including removable expansion means (500).
